# EUROPEAN PATENT APPLICATION

(11) **EP 3 970 778 A1**
(43) Date of publication of application: **23.03.2022**
(21) Application number: 21193614.1
(22) Date of filing: 27.08.2021
(51) Int. Cl.: A61M 25/02, A61M 25/01

(54) **MEDICAL DRESSING**

(30) Priority: 02.09.2020 US 202063073532 P; 12.08.2021 US 202117400329
(71) Applicant: MedXScience LLC, Harrington Park, NJ 07640 (US)
(72) Inventor: Dr. Berth, Ulrike, Harrington Park, NJ New Jersey 07640 (US)
(74) Representative: Osha Liang

(57) **Abstract**

A medical dressing is provided. It includes a patch (100) for adhering to a skin of a patient, the patch (100) comprising a first ring (120) having an orifice configured to correspond with an opening in the skin surface when the patch is adhered to the skin surface, the first ring (120) being configured to elastically adapt in size to enable longitudinal movement of a procedural device (catheter) therethrough, and a sleeve (110) in sealing connection with the first ring (120) at a proximal portion of the sleeve. The sleeve (110) surrounds part of the length of the catheter. The sleeve (110) includes a second ring (125) at a distal portion, the second ring (125) comprising a semi-elastic material configured to grip and seal around the catheter and to maintain a position along the external length of the catheter.

## Description

### Field of the Disclosure

The present disclosure relates to medical dressings, and more particularly, to a medical dressing device intended to create a sterile, closed-system coverage unit for natural and/or artificial openings in the body of a patient.

### Background of the Disclosure

When body openings are accessed or created for purposes of performing medical procedures within the body, it becomes important to maintain cleanliness and sterility in and around the area of the opening to avoid infection and cross contamination with bodily fluids, foreign and/or environmental contaminants, and debris.

The insertion, positioning, and removal of procedural devices (e.g. catheters) can be part of certain medical procedures for which either an artificial or natural body opening may be used to enable the catheter entry into the patient's body. During such procedures, a catheter, for example, may be inserted and removed, repositioned multiple times and in multiple directions with respect to the body opening, or stays in place after insertion. For example, during utilization of a Foley-type catheter for urinary relief, the catheter may be inserted to drain urine and is then removed and reinserted once urine needs to be drained again, or stays in place for continued urine drainage, for example in a patient with a neo-bladder or suprapubic urine catheter.

In some cases, the insertion, repositioning, and removal of a catheter or other procedural device through a body opening, may be performed at the home of a patient by, for example, a home-care provider or even the patient. Risk of fluid infiltration and/or infection may increase during such procedures. Further, in some cases, multiple body openings in near proximity may be present in the patient's body, which can cause cross contamination, especially in the situation were bodily fluids, for example urine and/ or stool is involved. Cross contamination can cause systemic infections, for example, urinary tract infection due to E.coli contamination. Ensuring that substances from one opening do not infiltrate or otherwise contaminate the other openings may be important in such cases.

US 2012/0232489 discloses a sealed sterile catheter dressing.

### SUMMARY OF THE DISCLOSURE

The present inventor has recognized that during certain medical procedures it is possible that bodily substances (e.g., fluids,) either from the body cavity or the procedural device being inserted, may "leak" or seep from the body opening, thereby potentially contaminating the surrounding area. It is further possible that insertion, reinsertion and/or repositioning of the procedural device, particularly in the presence of other body openings, may result in substance infiltration back into the body cavity.

The present inventor has thus determined that it is desirable to improve isolation of the opening for greater sterility and patient safety, among others.

According to embodiments of the present invention a medical dressing is provided. The medical dressing includes a patch configured for adhering to a skin surface of a patient, the patch including a first ring formed unitarily of an elastic material, the first ring having an orifice configured to correspond with an opening in the skin surface when the patch is adhered to the skin surface, the first ring being configured to elastically adapt in size to enable longitudinal movement of a procedural device therethrough while maintaining contact with the procedural device around a periphery of the procedural device, and a sleeve in sealing connection with the first ring at a proximal portion of the sleeve, the sleeve being configured to surround the procedural device over an external length of the procedural device, and comprising a second ring at a distal portion, the second ring comprising a semi-elastic material configured to grip the procedural device to seal about the periphery of the procedural device and to maintain a position along the external length of the procedural device.

By providing a medical dressing as described above, it becomes possible to more securely seal the area around a body opening used for insertion of a procedural device and thereby, to prevent contamination of the surrounding areas with fluids or other substances originating from the body cavity. Moreover, based on the presence of the first ring, fluids and substances from inside the body cavity may be "wiped" from an exterior of the procedural device as the procedural device exits the body opening, thereby further reducing risk of fluid/substance contamination of areas surrounding the opening.

The procedural device may form part of an indwelling catheter of the Foley type.

The first ring may be embedded in the patch.

The first ring may be adhered to a surface of the patch.

The sleeve may be seamless.

The sleeve may be formed unitarily with the first ring.

According to some embodiments, the sleeve, first ring, and at least one layer of the patch may be formed unitarily and without seams. Such a configuration may further ensure that surrounding areas are not contaminated by fluids/substances originating from within the body cavity or allow their spread to other body cavities, especially such cavities that are in close proximity to one another.

The first ring may include a locking ring configured to receive a complementary locking ring of the sleeve to enable removable connection of the sleeve with the first ring.

The second ring may be configured to adapt in size to seal about a periphery of any one of a plurality of procedural devices having different diameters within a predetermined range.

The predetermined range may be determined based on a procedural device type. For example, for a Foley catheter, a range may be from 4Fr to 26Fr (1.35 - 8.7mm), for a chest tube catheter, a range may be from 6Fr to 40Fr (2 - 13.33mm), for a suprapubic catheter, a range may be from 14Fr to 20Fr (4.7 - 6.7mm), for a dialysis catheter, a range may be from 8Fr to 13.5Fr (2.7 - 4.5mm), for a PICC line, a range may be from 4Fr to 6Fr (1.35 - 2mm), for a Swan -Ganz catheter, a range may be from 5Fr to 7.5Fr (1.67 - 2.5mm), for a central-line catheter, a range may be from 3Fr to 12Fr (1 - 4mm), and for a nephrostomy tube, a range may be from 5 Fr to 32 Fr (1.67 - 10.7mm).

The procedural device may be a multi-lumen type catheter.

The multi-lumen type catheter may include one of a Swan-Ganz catheter type and a central line catheter type.

The sleeve may define an internal volume through which the procedural device passes.

The sleeve may comprise a bacteriostatic coating on at least a portion of a surface of the internal volume and/or an external surface of the sleeve.

The removable connection is selected from among a snap-fit, a bayonet, a threaded connection, and a Luer-type connection, preferably the threaded connection, such as, for example, a screw-type connection.

The maintained position may be a predetermined position corresponding to a full extension length of the sleeve when a distal portion of the procedural device has breached the body opening. The predetermined position may also correspond to other lengths of the sleeve that are not full extension, for example, half extension, quarter extension, etc.,

According to further embodiments of the present disclosure, a medical dressing is provided. The medical dressing includes adhering means for adhering the medical dressing to a skin surface of a patient, the adhering means including unitary sealing means comprising an orifice configured to correspond with an opening in the skin surface when the adhering means is adhered to the skin surface, the unitary sealing means being configured to elastically adapt in size to enable longitudinal movement of a procedural device therethrough while maintaining contact with the procedural device around a periphery of the procedural device, sealed volume defining means in sealing connection with the unitary sealing means, the sealed volume defining means being configured to surround the procedural device over an external length of the procedural device, and comprising a second unitary sealing means, the second unitary sealing means comprising a semi-elastic material configured to grip the procedural device to seal about the periphery of the procedural device and to maintain a position along the external length of the procedural device

By providing a medical dressing as described above, it becomes possible to more securely seal the area around a body opening and thereby, to prevent contamination of the surrounding areas with fluids or other substances originating from the body cavity and vise versa preventing introduction of bodily fluids and/or external contaminants into the body cavity. Moreover, based on the presence of the unitary sealing means, fluids and substances from inside the body cavity may be "wiped" from an exterior of the procedural device as the procedural device exits the body opening, thereby further reducing risk of fluid/substance contamination of areas surrounding the opening.

The procedural device may be part of an indwelling catheter of the Foley type.

The unitary sealing means may be embedded in the adhering means.

The unitary sealing means may be adhered to a surface of the adhering means.

The sealed volume defining means may be formed seamlessly.

The sealed volume defining means may be formed unitarily with the unitary sealing means.

The unitary sealing means may include locking means configured to engage second locking means associated with the sealed volume defining means to enable removable connection of the sealed volume defining means and the unitary sealing means.

The second unitary sealing means may be configured to adapt in size to seal about a periphery of any one of a plurality of procedural devices having different diameters within a predetermined range.

The predetermined range may be determined based on a procedural device type, and is selected for example, for a Foley catheter, from 4Fr to 26Fr (1.35 - 8.7mm), for a chest tube catheter, from 6Fr to 40Fr (2 - 13.33mm), for a suprapubic catheter, from 14Fr to 20Fr (4.7 - 6.7mm), for a dialysis catheter, from 8Fr to 13.5Fr (2.7 - 4.5mm), for a PICC line, from 4Fr to 6Fr (1.35 - 2mm), for a Swan -Ganz catheter, from 5Fr to 7.5Fr (1.67 - 2.5mm), for a central-line catheter, from 3Fr to 12Fr (1 - 4mm), and for a nephrostomy tube, from 5 Fr to 32 Fr (1.67 - 10.7mm).

The procedural device may be a multi-lumen type catheter tube.

The multi-lumen type catheter tube may be one of a Swan-Ganz catheter type and a central line catheter type.

The sealed volume defining means may define an internal volume through which the procedural device passes.

The sealed volume defining means may include a bacteriostatic coating on at least a portion of a surface of the internal volume and/or an exterior surface of the sealed volume defining means.

The removable connection may be one of a snap-fit, a threaded connection, a bayonet, and a Luer-type connection, preferably the threaded connection.

The maintained position may be a predetermined position corresponding to a full extension length of the sleeve when a distal portion of the procedural device has breached the body opening.

It is intended that combinations of the above-described elements and those within the specification may be made, except where otherwise contradictory.

It is to be understood that both the foregoing general description and the following detailed description are exemplary and explanatory only and are not restrictive of the disclosure, as claimed.

The accompanying drawings, which are incorporated in and constitute a part of this specification, illustrate embodiments of the disclosure and together with the description, serve to explain the principles thereof.

### BRIEF DESCRIPTION OF THE DRAWINGS

Fig. 1A is a schematic depiction of an exemplary medical dressing according to embodiments of the present disclosure;
Fig. 1B shows the medical dressing of Fig. 1A with an exemplary sleeve portion in a compressed state;

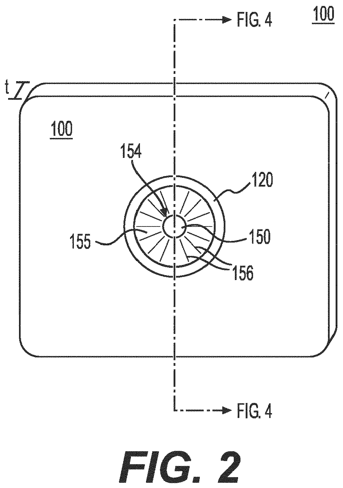

Fig. 2 is a detail of a patch of the exemplary medical dressing of Fig. 1A;
Fig. 3 shows an exemplary configuration for an adhesive connection between the ring and the sleeve according to some embodiments of the present disclosure;
Fig. 4A shows an exemplary configuration for a sleeve and ring in relation to the patch according to some embodiments of the present disclosure;
Fig. 4B shows another exemplary configuration for a sleeve and ring in relation to the patch according to some embodiments of the present disclosure;
Fig. 4C shows yet another exemplary configuration for a sleeve and ring in relation to the patch according to some embodiments of the present disclosure;
Fig. 5 shows an exemplary configuration for a locking mechanism between the ring and the sleeve according to some embodiments of the present disclosure;
Fig. 6 shows another exemplary configuration for a locking mechanism between the ring and the sleeve; and
Fig. 7 shows a detailed view of the locking mechanism shown at Fig. 6.

### DESCRIPTION OF THE EMBODIMENTS

Reference will now be made in detail to the present exemplary embodiments of the disclosure, examples of which are illustrated in the accompanying drawings. Wherever possible, the same reference numbers will be used throughout the drawings to refer to the same or like parts.

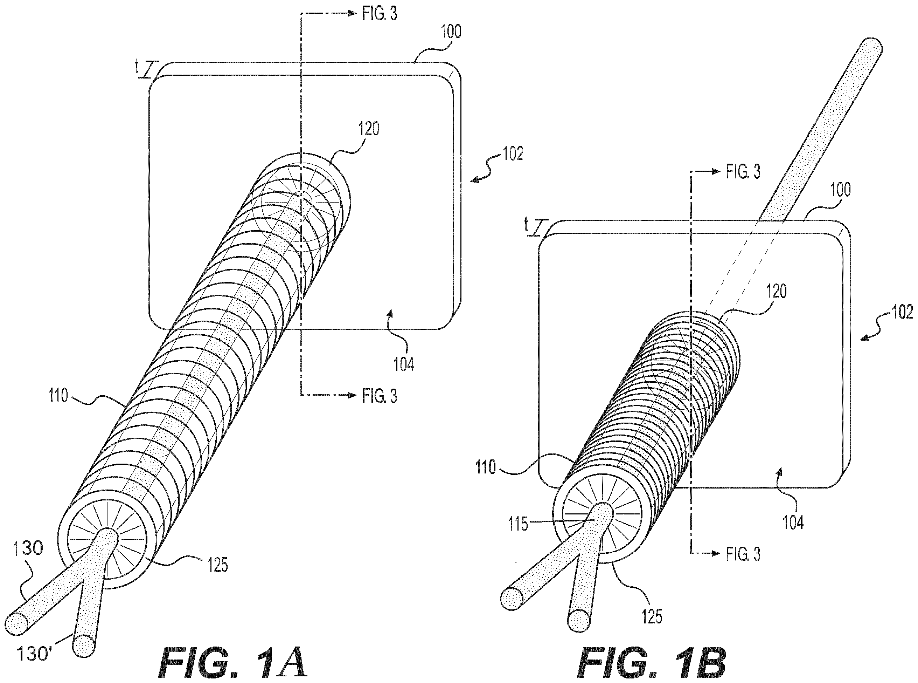

Fig. 1A is schematic depiction of an exemplary medical dressing according to embodiments of the present disclosure. The medical dressing is configured to be attached to the body of a patient in and around an area surrounding an opening in the body of the patient (referred to as the "access site") and for enabling passage of a procedural device 115 (e.g., a catheter) therethrough and into the body of the patient via the opening. The medical dressing includes, for example, a patch 100 (corresponding to an adhering means), a wiper ring 120 (corresponding to a first unitary sealing means), a sleeve 110 (corresponding to sealed volume defining means), and a locking ring 125 (corresponding to second unitary sealing means), among others. While patch 100 is shown as rectangular in the figures of the present disclosure, this shape is not intended to be limiting, and patch 100 may be of any suitable shape (e.g., circular, oval, rectangular, trapezoidal, etc.)

According to further embodiments, a biocidal/antiseptic wiping ring (not shown) may be provided on the dressing and/or a dressing coupler ring (not shown), while an additional distal locking ring (not shown) may be attached to a compatible coupler of the sleeve 110. A proximal sealing ring (not shown) may also be provided at a proximal end of the sleeve 110, this sealing ring being exposed to the external environment and preventing contaminants from entering the sleeve. For example, wiper ring 120 may be considered to be native with the patch 100, with a sponge (e.g., a biocidal sponge) placed adjacent to the wiper ring 120, away from an adhesive portion of the patch 100. A distal locking ring (e.g., a threaded coupler, such as, for example, a screw-on-type connector) may then be positioned adjacent to the sponge in a direction moving away from a surface of the patient's skin. One of skill will recognize that other ordering may be implemented without departing from the scope of the present disclosure. For example, the sponge may be considered native with the patch 100, while the wiper ring 120 may be provided adjacent the sponge moving further from the adhesive of the patch 100. Alternatively, the sponge may take the place of wiper ring 120 with no additional ring provided.

Procedural device 115 may comprise any suitable device for insertion into and removal from a body cavity, such as, for example, catheters, lines, and/or tubes. For example, procedural device 115 may comprise a catheter, e.g., a Foley catheter, a chest tube catheter, a suprapubic catheter, a dialysis catheter, a PICC line, a Swan-Ganz catheter, a central-line catheter, and/or a nephrostomy tube.

Procedural device 115 may have one or more lumen 130 at an end designed to remain outside the body. For example, procedural device may have one, two, three, four, five, or more converging lumens 130 for entry into a body cavity, e.g., by way of medical dressing according to embodiments of the present disclosure. While some figures of the present disclosure show a number of lumen 130 (e.g., lumen 130 and lumen 130'), this is exemplary for explanation, and not intended to be limiting.

According to some embodiments, the procedural device 115 may comprise a single or an array of light emitting diodes and/or lasers, for example, encased within material of the procedural device 115 as a native device and/or as an attachable piece configured for attachment to the procedural device. The array may be configured to apply a substantial level of light of desired wavelengths, and dosage (e.g., 400-500 nm for blue light and 220-290 nm for UVC) to the procedural device 115 and/or sleeve 110 (e.g., at a distal portion).

In such a configuration the material of the procedural device 115 may be configured to act as a light transmitting device (e.g., a light pipe) to carry the light to a tip or other desired area of the procedural device 115 when inserted into the body of the patient. With a sufficiently high power level, as discussed above, the light may radiate from the procedural device 115 to treat the internal and external surfaces for biocidal effects. This may help reduce biofilm formation of the internal lumen. In such an embodiment, power may be provided by any suitable means, e.g., battery power.

Patch 100 is formed of one or more layers, for example, a substrate layer, an adhesive layer, and a facing layer. Patch 100 may include a contact portion 102 configured for contact with the skin (e.g., via an adhesive layer) and a facing portion 104 located opposite and superposed on contact portion 102, and providing protection from elements exterior to the dressing. Patch 100 and its associated one or more layers, may be fabricated from any suitable material for use in medical applications, for example, a woven material, a nonwoven, polymeric, composite, etc.

According to some embodiments, a single layer of woven material may be implemented as patch 100. Fibers comprising the woven and/or non-woven material may be natural or synthetic, and may comprise, for example, of cotton, polypropylene, hydrogel, hydrofiber, alginate etc.

According to some embodiments, multiple layers of material (e.g., a first layer of woven, and a second layer of a non-woven material) may be implemented as patch 100. Alternatively, or in addition, patch 100 may comprise one or more polymeric layers, for example, providing a leak-proof membrane on a surface of facing portion 104. One of skill in the art will recognize upon review of the present disclosure, that the number of layers, and materials selected for implementation of patch 100 may depend upon an application for which the medical dressing is to be used, and the desired protection for the areas of a patient surrounding where patch 100 is to be adhered.

Materials comprising patch 100 may be opaque, translucent, or transparent, based on the material and treatments thereto. By providing a translucent or transparent material, visibility to a body opening may be improved, for example, during manipulation of a procedural device 115 that may be passed through an orifice in patch 100 into the body opening.

According to some embodiments, patch 100 may be hypoallergenic and/or latex free.

The material comprising patch 100 may also include one or more gels or other substances, for example, antibacterial substances, to aid in sterility of the area surrounding and covered by the patch 100 when patch 100 is applied to the skin surface. Such substances may be impregnated within the substrate material, coated on the material, or combinations thereof. Bactericides can include chlorhexidine, silver sulfadiazine, type coatings and/or a variety of antibiotics (e.g., gentamycin, bacitracin, etc.)

One or more adhesive layers may be included on contact portion 102 of patch 100, the adhesive comprising any suitable adhesive configured for medical use and for adhering patch 100 via contact portion 102 to a skin surface of a patient. For example, a pressure sensitive adhesive (e.g., acrylates, silicone, hydrogels, hydrocolloids or poly-urethanes) may be applied over at least a portion of contact portion 102 or over an entire surface of contact portion 102 configured to adhere to a skin surface, and the covered with a removable protective material to prevent contamination of the adhesive layer(s).

A thickness t comprising all implemented layers of patch 100 may range from 1 mm to about 10 mm, and according to some embodiments, 2 mm to 5 mm. One of skill will understand that such ranges are exemplary only, and not intended to be limiting.

Patch 100 may comprise a through-hole 107 (i.e., an orifice) permitting passage of a procedural device 115 (e.g. a catheter) through an entire thickness t of patch 100 (i.e., through facing portion 104, contact portion 102, and any adhesive layers.) The through hole may be formed in the layers of patch 100 by any suitable method, for example, punching, cutting, etc., and may be positioned on patch 100 to facilitate passage of procedural device 115 into the body opening, while maximizing sterility of surrounding areas. For example, through-hole 107 may be laser cut or punched centrally on facing portion 104 with respect to a periphery of patch 100. Other positions for through-hole 107 may be implemented without departing from the scope of the present disclosure.

When patch 100 is applied to a skin surface of a patient, through-hole 107 may be configured to be superimposed upon an opening in the skin surface. Therefore, through-hole 107 may be sized to enable at least partial, and even full access to an entire area of the opening in the skin through the hole 107. Such size may depend upon procedural device 115 to be inserted, and a diameter of through-hole 107 may range between about 4Fr to about 40Fr (particularly, about 4Fr to about 30Fr.) One of skill understands that 1Fr is equal to approximately 0.33 mm.

According to embodiments where the dressing is intended to provide anchoring for a removable system, attachment systems may be provided, for example, Luer-lock or rotational type systems (e.g., threads). Additionally, or alternatively, a groove and channel system may be provided allowing sliding of the sleeve 110 laterally onto the dressing. Cam and/or lever lock systems may also be implemented with the sleeve being configured to fit into an index point on the dressing. Once in place, a lever with a cam, for example, a flip-over tab, may be actuated to lock the sleeve 110 into place.

Wiper ring 120 is formed of an elastic material and is configured to elastically adapt in size to enable longitudinal movement of a procedural device 115 (e.g., a catheter) passing therethrough while a radially-inner surface (e.g., a lip or inner rim) of wiper ring 120 maintains contact with procedural device 115 around a periphery of procedural device 115.

Wiper ring 120 may be fabricated from any suitable, elastic material for example, rubber (e.g., neoprene rubber), thermoplastic elastomers (TPE), elastomeric silicone, sponge, etc.

Wiper ring 120 comprises a supporting portion 153, a membrane 155, and an orifice 150 comprising a lip 154. Importantly, components of wiper ring 120 are unitarily formed such that wiper ring 120 is a monolithic element to minimize risk of leakage from one side of wiper ring 120 to the other.

Wiper ring 120 may be formed via any suitable process for monolithically forming wiper ring 120, for example, by injection molding, thermoforming, compression molding, etc. One of skill in the art will recognize that these methods are merely exemplary, and other methods may be used for monolithically forming wiper ring 120 (e.g., stamping and/or cutting.)

Supporting portion 153 may be comprised by an outer periphery of wiper ring 120 and may be formed by a portion having a thickness greater than a thickness of membrane 155, for example.

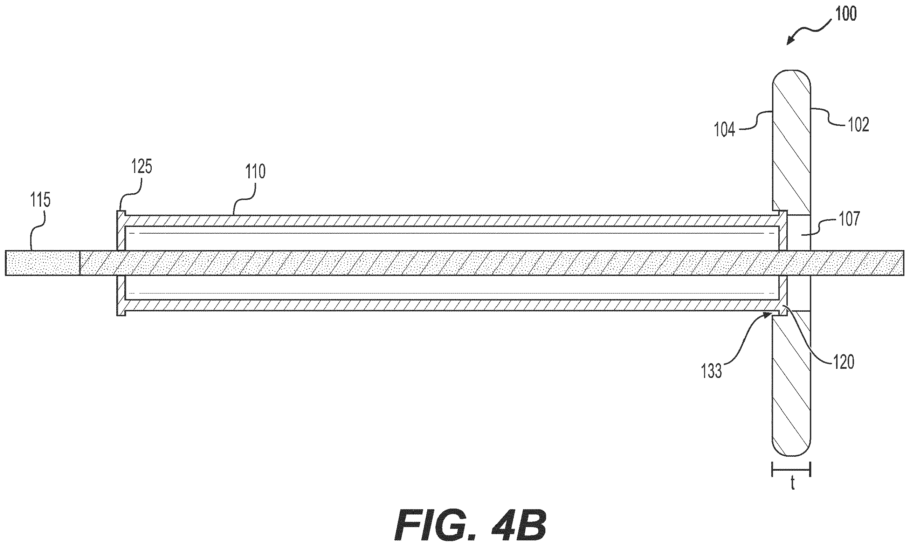

According to some embodiments, supporting portion 153 may be provided with additional support, for example, an additional loop of material embedded within supporting portion formed of for example, metal material and/or additional polymer materials about the periphery of wiper ring 120. Such support may be configured to provide additional rigidity and/or an interference type fit for wiper ring 120 when inserted into through-hole 107, as will be described below with regard to Fig. 4B.

Membrane 155 may extend radially inward from supporting portion 153 of wiper ring 120 covering an entire surface area between a lip 154 formed at orifice 150 and supporting portion 153 of wiper ring 120.

To provide a desired level of flexibility, membrane 155 may be formed with a desired thickness relative to supporting portion 153, based on, for example, a desired level of flexibility of membrane 155. According to some embodiments, a thickness reduction may be implemented, for example a 20-80 percent reduction in membrane 155 thickness relative to a thickness of supporting portion 153.

According to yet other embodiments, a thickness of membrane 155 may increase or remain constant relative to supporting portion 153. For example, a toroidal (e.g., a circular torus) or semi-toroidal shape (similar to a doughnut or grommet shape) may be implemented to form wiper ring 120. When referring to a "toroidal" or "semi-toroidal" shape in the context of the present disclosure, it is to be understood that any shape for a surface of rotation may implemented (e.g., a circular torus, a rectangular toroid, etc.), and that any such shape is intended to fall within the scope of the present disclosure.

In such embodiments, distance from the axis of revolution of the toroidal or semi-toroidal structure may be set based on the size of a procedural device 115 intended to be passed through wiper ring 120. A thickness increase may range from 20 to 80 percent relative to supporting portion 153, and may depend on a desired wiping effect to be obtained, and/or on a rigidity of a procedural device to be passed therethrough.

Membrane 155 may be provided with flexibility modifying features 156 such as, for example, undulations, lines of reduced thickness, and/or ribbing, etc. Implementation of such flexibility modifying features 156 may be based on desired flexibility and wiping capabilities of wiper ring 120.

Membrane 155 terminates at lip 154 forming orifice 150 within wiper ring 120. Orifice 150 is configured such that when the contact portion 102 of patch 100 is adhered to a skin surface of a patient, orifice 150, through-hole 107, and an opening in the skin surface, are substantially concentric to allow insertion of a procedural device 115 therethrough and facilitate entry into the body cavity.

A diameter of orifice 154 may be set based on a size of procedural device 115 intended to be inserted therethrough. For example, for a Foley catheter, a range may be from 4Fr to 26Fr (1.35 - 8.7mm), for a chest tube catheter, a range may be from 6Fr to 40Fr (2 - 13.33mm), for a suprapubic catheter, a range may be from 14Fr to 20Fr (4.7 - 6.7mm), for a dialysis catheter, a range may be from 8Fr to 13.5Fr (2.7 - 4.5mm), for a PICC line, a range may be from 4Fr to 6Fr (1.35 - 2mm), for a Swan -Ganz catheter, a range may be from 5Fr to 7.5Fr (1.67 - 2.5mm), for a central-line catheter, a range may be from 3Fr to 12Fr (1 - 4mm), for a nephrostomy tube a range may be from 5 Fr to 32 Fr (1.67 - 10.7mm.)

The diameter selected for orifice 150, and hence, orifice 154, regardless of the type of procedural device 115, should be selected such that a seal is created between lip 154 and an outer periphery of procedural device 115 when procedural device is inserted through orifice 154.

Sleeve 110 is configured to extend from wiper ring 120 at its proximal end while surrounding a procedural device 115 over a variable external length of procedural device 115, with a distal end of sleeve 110 maintaining a position along procedural device 115 via locking ring 125. As used in the present discussion, "external length of procedural device 115" is intended to refer to any portion of procedural device 115 extending away from and beyond supporting portion 153 of wiper ring 120 along a direction moving from contact portion 102 to facing portion 104. In other words, when the medical dressing is applied to a skin surface of a patient, "external length" refers to the portion of procedural device 115 outside the body opening extending beyond wiper ring 120.

According to some embodiments, wiper ring 120 may be a sponge-like material, for example, a biocidal sponge. The sponge may comprise any material that can maintain hydration and rehydration over the period of use of the procedural device 115. Such a configuration may help to reduce or eliminate contamination of the procedural device 115 prior to insertion allow for maintaining cleanliness of an access site long term.

According to some embodiments, the wiper ring 120 and the sponge-like material may both be provided separately within or near through-hole 107 of the patch 100.

An illustrative sponge may comprise a donut-style sponge configured for placement at the dressing level above the access site. In such a configuration, the diameter of the inner sponge hole is configured smaller than a diameter of the procedural device 115.

The sponge may be configured as a solid component or as a clamp configured to clamp around an exterior surface of the procedural device 115. The sponge may be replaceable or permanent (i.e., for the useful life of the medical dressing), and where permanent, any suitable method for re-hydration (e.g., via an access point) may be implemented.

The sponge can be hydrated with a cleaning substance, e.g., a liquid surfactant and/or biocidal material, that is safe for skin contact (i.e., without harmful side effects) and safe to pass into the access site of the patient. Such material can coat the procedural device 115 as it is inserted, aiding in immediate removal of bacteria, and possibly coating the device 115 long term to fight against formation of biofilm.

In practice, the procedural device 115 may be inserted through a hole in the middle of the sponge and/or the wiper ring 120, and as the procedural device 115 is passed through, the smaller diameter of the inner sponge hole can mechanically rub against an external surface of the procedural device 115, acting to remove foreign debris.

According to another example, a removable sheath extending over a length of the procedural device 115 may be implemented. Such a sheath may remain in place or be removed after insertion of the procedural device 115.

Sleeve 110 may be seamless along its longitudinal length and may correspond substantially in cross section to a shape of wiper ring 20, e.g., circular in cross section and extending as a cylinder along its longitudinal axis. Depending on a shape of wiper ring 120, sleeve 110 may have other cross-sectional shapes without departing from the scope of the present disclosure (e.g., rectangular, oval, etc.)

Sleeve 110 may comprise material that is tear resistant and extendible and/or compressible such that an apparent length of sleeve 110 may vary relative to patch 100. Such features may serve to ensure a variable, leak-proof inner volume is defined within sleeve 110, thereby allowing procedural device to extend and be moved therethrough.

Fig. 1B shows the medical dressing of Fig. 1A with an exemplary sleeve 110 in a compressed state (i.e., shortened state,) while Fig. 1A shows the sleeve in the extended state (i.e., lengthened state.) Sleeve 110 is configured to allow movement of the procedural device 115 into and out of the body opening via wiper ring 120 and through-hole 107 while a distal portion of sleeve 110 maintains a longitudinal position along an external portion of procedural device 115 by way of locking ring 125. In other words, as procedural device 115 is inserted further into a body cavity, sleeve 110 is configured to "compress" or shorten its apparent length in conjunction with movement of procedural device 115, thereby decreasing an internal volume created around procedural device 115 by sleeve 110. Likewise, as procedural device 115 is extracted from a body cavity, sleeve 110 is configured to "extend" or increase its apparent length relative to patch 100 in conjunction with movement of procedural device 115, thereby increasing an internal volume created around procedural device 115 by sleeve 110.

According to some embodiments, extension and compression of sleeve 110 and manipulation of the internal volume created thereby, may be accomplished by pleating of sleeve 110 to enable a "bellows-style" back-and-forth folding of sleeve 110 during movement of procedural device 115 (e.g., also known as accordion folds.) Pleating formed in sleeve 110 may be of any suitable size to allow for desirable extension and compression.

In another example, extension and compression of sleeve 110 and manipulation of the internal volume created thereby, may be enabled via the elasticity of the material forming sleeve 110, for example, or even crumpling of the material forming sleeve 110. Compression and extension of sleeve 110 may also be enabled via combinations of elasticity, crumpling, pleating, etc., within departing from the scope of the present disclosure.

Sleeve 110 may comprise one or more compounds configured to provide antibacterial/biocidal properties. For example, one or more layers of an antimicrobial compound (e.g., titanium dioxide) may be present on one or more surfaces (e.g., inner and outer) of the sleeve 110. The antimicrobial compound may be inactive or partially active until external energy is applied, for example, light energy. The dressing including the sleeve 110 may then be periodically exposed to one or more wavelengths of light (e.g., UV, blue, etc.) to cause the compound (e.g., titanium dioxide) to act in an antimicrobial manner. For example, a light wand or other suitable light emitting device may be introduced near or within the sleeve 110 and illuminated to provide the desired light energy.

According to another example, the sleeve 110 and/or other portions of the dressing (e.g., wiper ring 120, locking ring 125, etc.) may be provided with one or more layers of antibacterial/antimicrobial material as desired. The antibacterial/antimicrobial material may comprise, for example, chlorhexidine, silver sulfadiazine, type coatings and/or a variety of antibiotics (e.g., gentamycin, bacitracin, etc.), similar to those noted above.

Locking ring 125 is joined to and may be sealed with a distal portion of sleeve 110 (i.e., portion of sleeve 110 furthest from patch 100) and is configured to adjustably grip procedural device 115 to maintain a desired longitudinal position along procedural device 115 thereby allowing sleeve 110 to move in conjunction with movement of procedural device 115.

According to some embodiments, locking ring 125 may be similar or even identical in configuration to wiper ring 120 and may therefore provide additional sealing and wiping functions at the proximal end of sleeve 110, once the lock is released and procedural device able to move freely through locking ring 125.

Locking ring 125 may be configured such that a light interference fit between an external periphery of procedural device 115 and an inner orifice of locking ring 125, thereby enabling sufficient force to maintain sleeve 110 at a position along a length of procedural device 115 during a medical procedure. Following completion of the medical procedure, an operator may exert sufficient force to overcome the interference fit, thereby allowing the procedural device to be pulled through locking ring 125.

Alternatively, or in addition, various locking mechanisms may be implemented with regard to locking ring 125 to enable fixing of locking ring 125 at a position along procedural device 115. For example, one or more clamping devices may be implemented, such as, hose clamps, tubing clamps, quick fit clamps, etc. thereby enabling an operator to adjust a level of force applied to procedural device 115 to maintain sleeve 110 thereon. Any suitable fixing device enabling locking ring 125 to maintain its position along may be implemented without departing from the scope of the present disclosure.

A distal portion of sleeve 110 and wiper ring 120 may be joined in any suitable manner so as to provide a fluid tight seal between the distal portion of sleeve 110 and wiper ring 120.

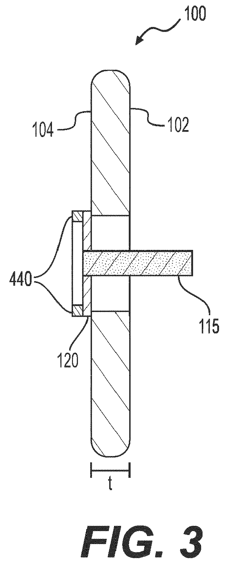

Fig. 3 shows an exemplary configuration for an adhesive connection between wiper ring 120 and sleeve 110 according to some embodiments of the present disclosure. As shown at Fig. 3, a layer of adhesive 440 may be provided around a periphery of supporting portion 153 of ring 120, such that a distal portion of sleeve 110 may be adhered to and sealed with ring 120. One of skill in the art will recognize that any suitable adhesive may be used without departing from the scope of the present invention.

In such a configuration, sleeve 110 may include a supporting portion (not shown) about its distal end facilitating adhesion with wiper ring 120. Supporting portion of sleeve 110 may comprise an increased thickness and/or width in the material comprising sleeve 110 at the distal end of sleeve 110. Other configurations for facilitating adhesion in such embodiments are also contemplated and intended to fall within the scope of the present disclosure, for example, a reinforcement ring having similar diameter to supporting portion 153 of wiper ring 120, the reinforcement ring being embedded in distal portion of sleeve 110.

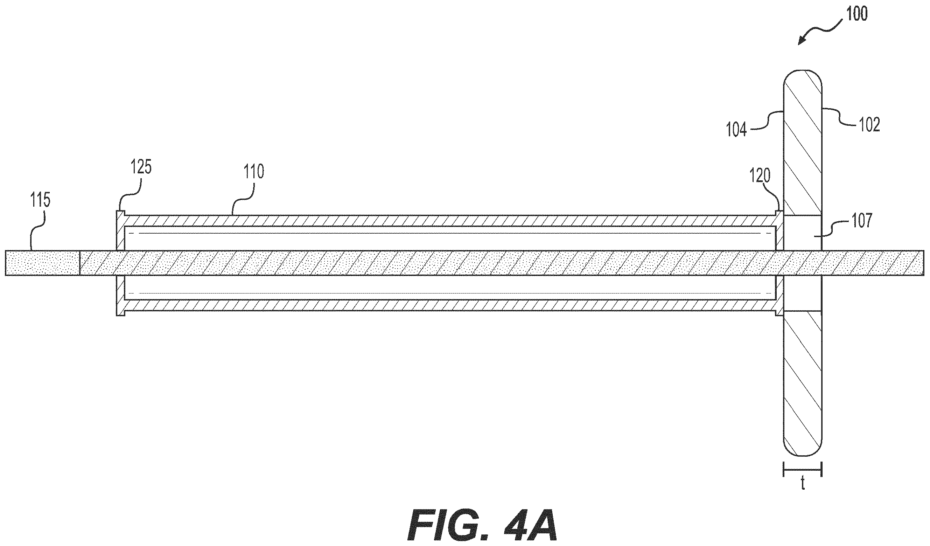

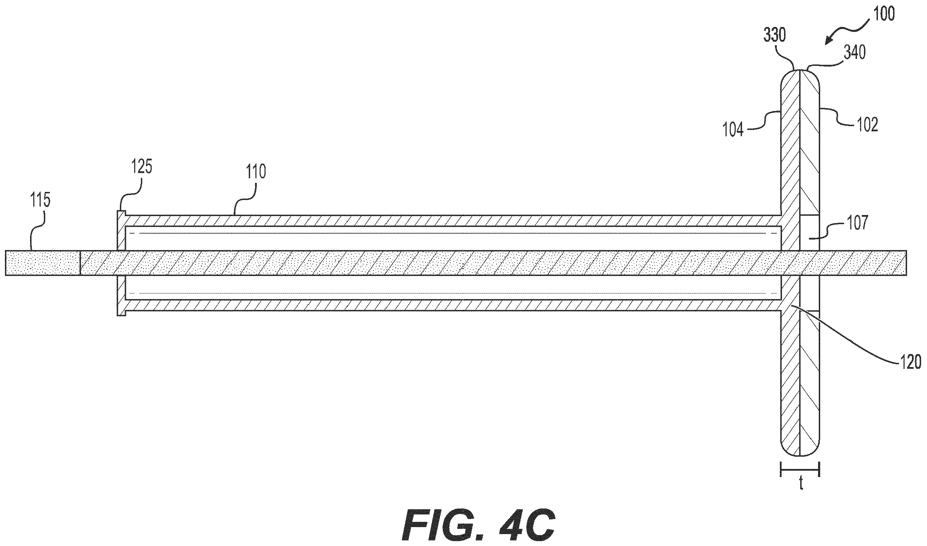

In order to maintain fluid tightness, it may be desirable to limit the number of seams in the medical dressing of the present disclosure. In Figures 4A-4C at least sleeve 110 and wiper ring 120 are formed unitarily resulting in a monolithic and seamless structure for at least sleeve 110 and ring 120. As shown at Fig. 4C, facing portion of patch 100 is also formed unitarily with sleeve 110 and ring 120, such that further improvements in seamlessness, fluid tightness, and leak prevention may be achieved.

Notably, locking ring 125 may optionally be formed unitarily with at least sleeve 110 and wiper ring 120. In addition, as shown at Fig. 4C, locking ring may be formed unitarily with the sleeve 110, wiper ring 120, and facing portion 104.

Figs. 4A-4C and 5-6 also highlight various methods by which wiper ring 120 can be sealingly joined with patch 100 resulting in substantially concentric alignment of an orifice of wiper ring 120, through-hole 107, and a body opening when patch 100 is applied to a skin surface, while also limiting leakage risk.

Fig. 4A shows an exemplary configuration for sleeve 110 and wiper ring 120 in relation to patch 100, where sleeve 110, wiper ring 120, and optionally locking ring 125, are unitarily fabricated. According to this embodiment, sleeve 110 and wiper ring 120 are formed monolithically and simultaneously from a desired material. Fabrication of unitary sleeve 110 and wiper ring 120 may be performed by, for example, injection molding, blow molding, extrusion, or any other suitable technique.

As shown at Fig. 4A the sleeve 110 unitarily formed with wiper ring 120 unit may be adhered to and sealed with facing portion of patch 100 such that patch 100 supports supporting portion 153 of wiper ring 120, and that orifice 154 of wiper ring 120 is substantially concentric with through-hole 107, and hence with an opening in a skin surface when patch 100 is adhered to the skin surface. Any suitable adhesive may be used for securing and sealing the unit to patch 100, for example, a silicone-, acrylates-, hydrogels-, hydrocolloids- or poly-urethanes adhesive, among others.

Fig. 4B shows another exemplary configuration for sleeve 110 and ring 120 in relation to the contact portion according to some embodiments of the present disclosure. As shown at Fig. 4B, a countersunk recess 133 may be formed in facing portion 104 of patch 100, the recess 133 being configured, for example, to produce an interference fit between supporting portion 123 of wiper ring 120 and layers of patch 100. In such embodiments, a sealant (e.g., silicone) may be provided around a periphery of supporting portion 153 of wiper ring 120, such that a seal is produced at the interference fit junction.

Alternatively, no interference fit may be created between supporting portion 123 of wiper ring 120, and any suitable sealant and/or adhesive may be used to create a seal and retain wiper ring 120 within patch 100.

Fig. 4C shows yet another exemplary configuration for sleeve 110 and ring 120 in relation to the contact portion according to some embodiments of the present disclosure. As shown at Fig. 4C, facing portion 104, wiper ring 120, sleeve 110, and optionally locking ring 125 may be formed unitarily from a desired material such that a single piece 330 may be adhered to a substrate 340 corresponding to contact portion 102 of patch 100. Any suitable adhesive, for example, silicone adhesive may be used to adhere and seal facing portion 104 with contact portion 102 in such an embodiment.

Alternatively, facing portion 104 and contact portion 102 may be formed unitarily with ring 120 and sleeve 110, such that a complete monolithic unit may result for the medical dressing. In such a case, an adhesive for adhering patch 100 to skin of patient may be applied to contact portion 102 of the unit, and subsequently adhered to the skin.

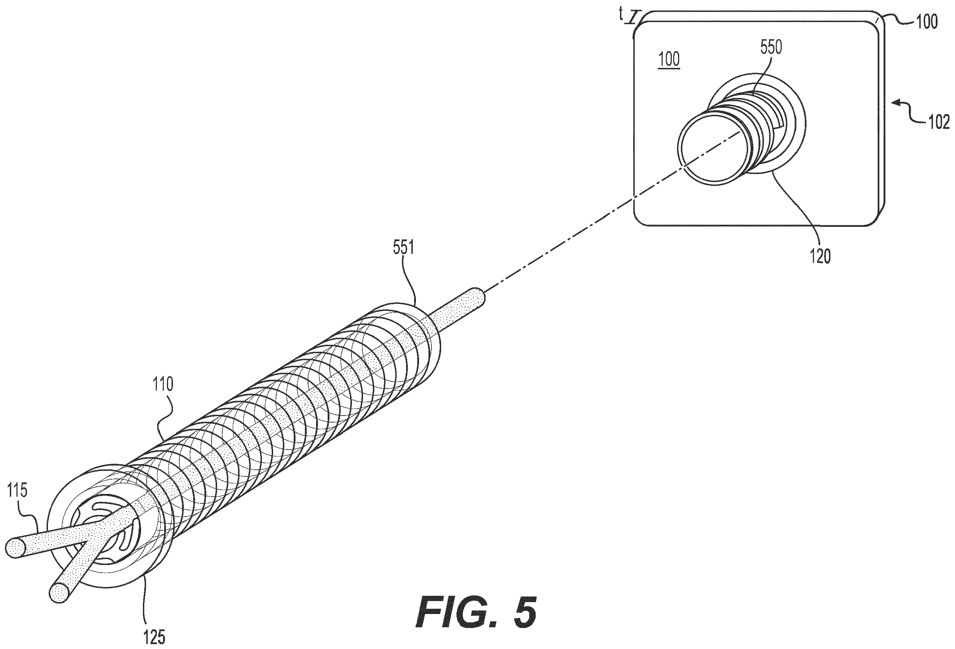

According to additional embodiments, a locking mechanism may be used for joining sleeve 110 and patch 100. Fig. 5 shows one exemplary configuration for a locking mechanism between the ring and the sleeve according to some embodiments of the present disclosure. In the example of Fig. 5, a Luer-lock type connection is provided, whereby a first threaded adapter 550 is sealingly affixed to patch 100, and a complementary adapter 551 is sealingly affixed to sleeve 110. Such a configuration may allow patch 100 to be affixed to the skin of a patient at a desired location, and subsequently, sleeve 110 threaded and tightened on to first threaded adapter 550 via second threaded adapter 551.

First and/or second threaded adapter 550 and 551 may be provided with an anti-rotational device configured to prevent loosening of the locking mechanism once sleeve 110 has been threaded and tightened on to patch 100. Such a device may comprise a spring-lock configured to block rotation once sleeve has been threaded on to first adapter 550 by a predetermined distance.

Notably, when considering a threaded-type connection, such a connection need not be of the Luer type, and any other suitable threaded connection (e.g., standard nut-and-bolt type threads) may be implemented without departing from the scope of the disclosure.

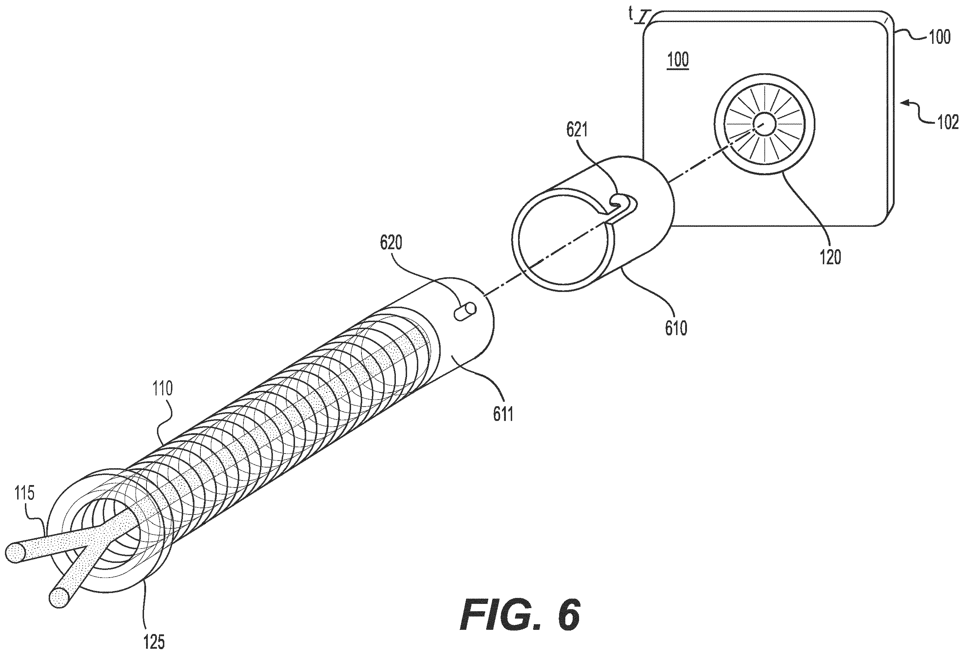

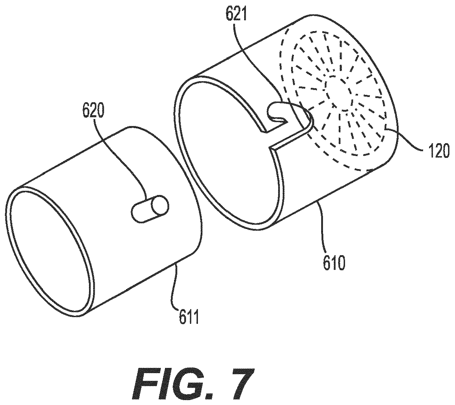

Figs. 6 and 7 provide views of another exemplary configuration for a locking mechanism between wiper ring 120 and sleeve 110 and take the form of a bayonet type connection. The bayonet type connection may comprise a ring adapter 610 sealingly affixed to patch 100 and a sleeve adapter 611 sealingly affixed to sleeve 110. Each of ring adapter 610 and sleeve adapter 611 may be formed unitarily with its corresponding part or may be affixed via any other suitable manner (e.g., adhesive). Notably, ring adapter 610 may be unitarily formed with facing portion 104, while sleeve adapter 611 is adhered to sleeve 110, and vice versa.

One or more pins 620 may be provided on a sleeve adapter 611 with a corresponding number of slots 621 provided on ring adapter 610. Sleeve adapter 611 may then be aligned with ring adapter 610 and inserted, such that upon twisting of sleeve adapter 620, the bayonet connection is created.

For the embodiments of Figs. 6-7, wiper ring 120 may be provided on patch 100, as shown in Fig. 6, or may be provided on adapter ring 610, as shown in Fig. 7. One of skill in the art will recognize that any desired placement of wiper ring 120 may be made (e.g., at contact portion 102 of patch 100) without departing from the scope of the present invention.

According to another embodiment (not shown,) a snap-fit connection may be implemented as a locking mechanism between sleeve 110 and wiper ring 120 or patch 100. In such an embodiment, once sleeve 110 has been pressed on to a corresponding locking portion on patch 100 by a specified distance, one or more biased devices may be forced into a receiving portion present on patch 100 such that removal of sleeve 110 is prevented.

Embodiments of the present disclosure may enable a high level of sterility to be maintained in and around one or more body opening through which procedural devices are intended to be inserted. Therefore, health benefits, such as limiting risk of cross contamination and infections, depending on catheter type to a patient undergoing one or more medical procedures involving insertion of such procedural devices can be improved while also minimizing patient discomfort. Such benefits can be especially helpful for patients using long-term, in-dwelling procedural devices (e.g., Foley catheters, suprapubic catheters, PICC Lines or Chest tube) and/or patients have multiple body openings in close proximity (e.g., for a plurality of different medical procedures.)

Moreover, by providing locking mechanisms allowing interchangeable sleeve/ring configurations to be implemented, multiple procedures may be carried out via one body opening without the need to change or modify a configuration of patch 100.

Modifications may be implemented to the presently disclosed design without departing from the scope of the present application. For example, a fluid wash chamber may be provided to allow intermittent washing of the external portions of the procedural device 115. According to such an example, a rigid or flexible pouch attached to the dressing as an additional sheath outside or inside the of the sleeve 110 may be provided, for example, at a distal end of the device beyond the locking ring 125. The sheath would feature at least one sphincter at the distal portion, providing a fluid-tight seal around the procedural device 115. The procedural device 115 may be placed through this sheath upon insertion. The sheath may also feature a coupling to attach a syringe or other method to introduce fluid into the sheath forming the fluid wash chamber. A biocidal or cleaning solution may be passed into the sheath to intermittently wash the procedural device 115. Following the washing operation, the fluid can be thereby withdrawn and/or the sheath removed until a next washing.

As another example, one or more pH sensitive layers may be provided on the patch 100 and/or other areas of the dressing in contact with fluids from the access site (e.g., the sleeve 110). The pH sensitive layers may be configured to react with any number of biomarkers, bacteria, and/or environmental factors to indicate change in the state of the dressing, patch 100, sleeve 110, procedural device 115, etc.

In addition, a temperature reactive material (e.g., a thermochromic fabric) and/or a temperature sensor may be provided with patch 100 such that tissue temperatures may be monitored via a change to signal a possible infection.

Feedback from such systems may include, for example, a change in substrate color based on detected changes in the access area. For example, where a thermochromic fabric changes color to indicate a rise in temperature, this may be interpreted as a possible sign of infection. Additionally, electrical or other reaction detection may be implemented through an electronic monitoring system (e.g., including a pH sensor, a temperature sensor, etc.), and upon determination of a marker (e.g., pH change), the system may alert the user, for example, through mobile system integration, an audible alarm, tactile feedback (e.g., vibration), etc.

Throughout the description, including the claims, the term "comprising a" should be understood as being synonymous with "comprising at least one" unless otherwise stated. In addition, any range set forth in the description, including the claims should be understood as including its end value(s) unless otherwise stated. Specific values for described elements should be understood to be within accepted manufacturing or industry tolerances known to one of skill in the art, and any use of the terms "substantially" and/or "approximately" and/or "generally" should be understood to mean falling within such accepted tolerances.

Where any standards of national, international, or other standards body are referenced (e.g., ISO, etc.), such references are intended to refer to the standard as defined by the national or international standards body as of the priority date of the present specification. Any subsequent substantive changes to such standards are not intended to modify the scope and/or definitions of the present disclosure and/or claims.

It is intended that the specification and examples be considered as exemplary only, with a true scope of the disclosure being indicated by the following claims.

## Claims

1. A medical dressing, comprising:
adhering means (100) for adhering the medical dressing to a skin surface of a patient, the adhering means (100) comprising:
unitary sealing means (120) comprising an orifice (154) configured to correspond with an opening in the skin surface when the adhering means (100) is adhered to the skin surface, the unitary sealing means (120) being configured to elastically adapt in size to enable longitudinal movement of a procedural device (115) therethrough while maintaining contact with the procedural device (115) around a periphery of the procedural device (115);
sealed volume defining means (110) in sealing connection with the unitary sealing means (120), the sealed volume defining means (110) being configured to surround the procedural device (115) over an external length of the procedural device (115), and comprising a second unitary sealing means (125), the second unitary sealing means (125) comprising a semi-elastic material configured to grip the procedural device (115) to seal about the periphery of the procedural device (115) and to maintain a position along the external length of the procedural device (115).

2. The medical dressing according to claim 1, wherein the procedural device (115) is part of an indwelling catheter of the Foley type.

3. The medical dressing according to any of claims 1-2, wherein the unitary sealing means (120) is embedded in the adhering means (100).

4. The medical dressing according to any of claims 1-3, wherein the unitary sealing means (120) is adhered to a surface of the adhering means (100).

5. The medical dressing according to any of claims 1-4, wherein the sealed volume defining means (110) is formed seamlessly and unitarily with the unitary sealing means (120).

6. The medical dressing according to any of claims 1-4 wherein the unitary sealing means (120) includes locking means (550) configured to engage second locking means (551) associated with the sealed volume defining means (110) to enable removable connection of the sealed volume defining means (110) and the unitary sealing means (120).

7. The medical dressing according to any of claims 1-6, wherein the second unitary sealing means (125) is configured to adapt in size to seal about a periphery of any of a plurality of procedural devices (115) having different diameters within a predetermined range.

8. The medical dressing according to claim 7, wherein the predetermined range is determined based on a procedural device type, and is selected:
for a Foley catheter, a range may be from 4Fr to 26Fr;
for a chest tube catheter, a range may be from 6Fr to 40Fr;
for a suprapubic catheter, a range may be from 14Fr to 20Fr;
for a dialysis catheter, a range may be from 8Fr to 13.5Fr;
for a PICC line, a range may be from 4Fr to 6Fr;
for a Swan-Ganz ctheter, a range may be from 5Fr to 7.5Fr and
for a central-line catheter, a range may be from 3Fr to 12Fr; and
for a nephrostomy tube catheter, a range may be from may be from 5 Fr to 32 Fr.

9. The medical dressing according to any of claims 1-8, wherein the procedural device (115) is a multi-lumen type catheter.

10. The medical dressing according to claim 9, wherein the multi-lumen type catheter comprises one of a Swan-Ganz catheter type and a central line catheter type.

11. The medical dressing according to any of claims 1-10, wherein the sealed volume defining means (110) defines an internal volume through which the procedural device (115) passes.

12. The medical dressing according to claim 11, wherein the sealed volume defining means (110) comprises a bacteriostatic coating on at least a portion of a surface of the internal volume and/or an exterior surface of the sealed volume defining means (110).

13. The medical dressing according to claim 6, wherein the removable connection is one of a snap-fit, a bayonet, and a Luer-type connection, preferably the bayonet.

14. The medical dressing according to any of claims 1-13, wherein the maintained position is a predetermined position corresponding to a full extension length of the sleeve when a distal portion of the procedural device (115) has breached the body opening.
